# EUROPEAN PATENT APPLICATION

(11) **EP 1 955 685 A1**
(43) Date of publication of application: **13.08.2008**
(21) Application number: 06833218.8
(22) Date of filing: 24.11.2006
(51) Int. Cl.: A61J 1/00, A61M 39/02, A61M 5/00, A61M 5/36

(54) **CONNECTION ADAPTOR AND CONNECTION DEVICE FOR LIQUID DRUG**

(30) Priority: 25.11.2005 JP 2005340293
(71) Applicant: Nemoto Kyorindo Co., Ltd., Tokyo 113-0033 (JP)
(72) Inventor: NEMOTO, Toru, Tokyo 1130033 (JP); IKOMA, Tomoyuki, Tokyo 1130033 (JP); FUKUDA, Takashi, Tokyo 1130033 (JP)
(74) Representative: Cabinet Plasseraud
(86) International application number: PCT/JP2006/323414
(87) International publication number: WO 2007/061040

(57) **Abstract**

A connection adapter comprises a hollow supply needle communicating with a liquid supply tube connection portion, a hollow discharge needle communicating with a chemical liquid injection tube connection portion, a holder portion supporting the supply needle and the discharge needle, and a protection cap removably fit on the polder portion, the supply needle and the discharge needle being accommodated by and hermetically sealed in the protection cap and the holder portion. By using the adapter, a liquid is supplied from the liquid supply tube to remove air in the liquid supply tube and the chemical liquid injection tube, and then the protection cap is removed, and then the needles are inserted into a chemical liquid container having an opening sealed with an elastic member, and a push-out liquid is supplied from the liquid supply needle into the chemical liquid container, whereby the chemical liquid is injected through the discharge needle and the chemical liquid injection tube into a patient.

## Description

### Technical Field

The present invention relates to a connecting apparatus for connecting a tube to a chemical liquid container to inject a chemical liquid into a patient, and more particularly, to an apparatus for connecting a liquid supply tube and a chemical liquid injection tube to a chemical liquid container which contains a radioactive contrast medium.

### Background Art

Presently available imaging diagnostic apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses, angiography apparatuses, MRA (MR angiography) apparatuses and the like. Those apparatuses are often used in diagnoses with the aid of a contrast medium for the purpose of improving the accuracy of the diagnosis, and chemical liquid injectors for automatically performing the injection of the contrast medium have been put into practical use.

A chemical liquid containing a radioisotope is typically used as a contrast medium in tests performed with PET apparatuses or SPECT apparatuses. To prevent exposure to radiation, Japanese Patent Laid-Open No. 2004-290455 (Patent Document 1) describes injection of a chemical liquid contained in a liquid syringe on which a syringe cover made of tungsten is placed for blocking radiation, by way of example.

Since a radioisotope in a contrast medium decays over time in an RI test, a radioactive contrast medium is conventionally prepared and is filled into a syringe for use at a hospital where the contrast medium should be injected into a patient. In recent years, however, a rapid increase in number of hospitals where PET apparatuses are installed has resulted in establishment of a system for filling a radioactive contrast medium into a chemical liquid container and delivering the container to a hospital. Such a chemical liquid container is a cylindrical vial made of glass and has an opening at its end sealed with an elastic member made of silicone rubber or the like. The chemical liquid container is put in a shield container made of lead, tungsten or the like provided with a removable lid and is delivered to the hospital.

For filling the contrast medium into a liquid syringe from such a chemical liquid container, an operator removes the shield cap of the shield container which contains the chemical liquid container, inserts a needle of the liquid syringe into the exposed elastic member, and sucks the contrast medium into the liquid syringe from the chemical liquid container. Then, the operator replaces the needle of the liquid syringe with an extension tube and mounts the liquid syringe on an automatic injector to inject the radioactive contrast medium into a patient from the liquid syringe through the extension tube.

Patent Document 1: Japanese Patent Laid-Open No. 2004-290455

### Disclosure of the Invention

### Subject to be Solved by the Invention

The chemical liquid container which contains the radioactive contrast medium is sealed wholly with the shield container as described above, so that a leak of radioactivity to the surroundings is prevented. However, the operator is exposed to the radiation in the course of the suction of the contrast medium from the chemical liquid container into the liquid syringe and the mounting of the liquid syringe on the chemical liquid injector.

To solve the problem, the present applicant has developed and proposed a chemical liquid injection system capable of directly injecting a radioactive contrast medium into a patient from a chemical liquid container in WO2005/123161 (Japanese Patent Application No. 2004-182475) and WO2006/051855 (Japanese Patent Application No. 2005-81072), both of which were not opened to the public at the priority date of the present application. In these systems, a chemical liquid container is connected to a patient through a chemical liquid injection tube and a liquid supply tube is connected to the chemical liquid container to supply a liquid such as physiological saline to the chemical liquid container to inject the chemical liquid into the patient from the chemical liquid container. In this case, the leading end of the liquid supply tube and the trailing end of the chemical liquid injection tube which are connected to the chemical liquid container are made of needle members. The two needles are inserted into an elastic member of the chemical liquid container to allow the connection of the liquid supply tube and the chemical liquid injection tube to the chemical liquid container without manually opening the elastic member of the chemical liquid container. Especially, Japanese Patent Application No. 2005-81072 has provided an apparatus for inserting the two needles into the elastic member of the chemical liquid container to minimize exposure of the operator to radiation.

The present invention has been made to further improve the abovementioned apparatus for inserting the two needles into the elastic member of the chemical liquid container, and it is an object of the present invention to provide an apparatus for efficiently removing air from a liquid supply tube to the leading end of a chemical liquid injection tube (including a needle, a catheter and the like to be connected to a patient), and a protecting tool from a sharp needle member.

### Means to Solve the Subject

The present invention relates to the following.
1. A connection adapter comprising:
   a liquid supply tube connection portion;
   a chemical liquid injection tube connection portion;
   a hollow supply needle communicating with the liquid supply tube connection portion;
   a hollow discharge needle communicating with the chemical liquid injection tube connection portion;
   a holder portion supporting the supply needle and the discharge needle; and
   a protection cap removably fit on the holder portion, the supply needle and the discharge needle being accommodated by and hermetically sealed in the protection cap and the holder portion.
2. The connection adapter according to 1, wherein the connection adapter is used in a chemical liquid injection system in which the connection adapter is connected to a liquid supply tube and a chemical liquid injection tube with the protection cap being fit, and the connection adapter is supplied with a liquid from the liquid supply tube to remove air in the liquid supply tube and the chemical liquid injection tube, and then the protection cap is removed, and then the supply needle and the discharge needle are inserted into an elastic member of a chemical liquid container having an opening sealed with the elastic member and containing a chemical liquid, and a push-out liquid is supplied from the liquid supply tube into the chemical liquid container through the supply needle, whereby the chemical liquid in the chemical liquid container is discharged through the discharge needle, passes through the chemical liquid injection tube, and is injected into a patient.
3. The connection adapter according to 2, wherein the protection cap has a sheath shape elongated along the direction of the two needles and has an engaging portion formed in a part of the sheath shape.
4. A protection cap removing mechanism for the connection adapter according to any one of 1 to 3, comprising:
   a space portion opened upward and capable of accommodating a part of the protection cap from above; and
   an engaging mechanism engaging with the protection cap placed in the space portion and preventing an upward movement of the protection cap when the holder portion of the connection adapter is moved upward.
5. The protection cap removing mechanism according to 4, wherein the space portion has a cylindrical shape and has a notch formed in a wall thereof, the engaging mechanism which has n engaging piece is moved in a direction perpendicular to a direction in which the protection cap is inserted and capable of extending into the space portion.
6. A chemical liquid connecting apparatus comprising:
   the connection adapter according to any one of 1 to 3;
   a container holding mechanism holding the chemical liquid container such that the opening of the container is placed at the top;
   a holder moving mechanism vertically moving the connection adapter to an upper retraction position and a lower insertion position; and
   a container moving mechanism horizontally moving the container holding mechanism to an insertion position opposed to the retraction position of the connection adapter in the lower side and an initial position not opposed to the retraction position.
7. The chemical liquid connecting apparatus according to 6, further comprising the protection cap removing mechanism according to 4 or 5 and capable of being placed on the container moving mechanism.
8. The chemical liquid connecting apparatus according to 6 or 7, further comprising a container lifting mechanism vertically moving the chemical liquid container at the initial position.
9. The chemical liquid connecting apparatus according to 8, wherein the horizontal movement of the container moving mechanism is allowed at the position where the container lifting mechanism is lowered at the initial position.
10. The chemical liquid connecting apparatus according to any one of 6 to 9, wherein the chemical liquid is a contrast medium containing a radioactive source, and the chemical liquid container is housed in a shield container and is placed in the container holding mechanism with the elastic member of the chemical liquid container exposed at the top.
11. A chemical liquid injector comprising:
   the chemical liquid connecting apparatus according to any one of 6 to 10;
   a liquid supply tube connected to the chemical liquid connecting apparatus;
   a chemical liquid injection tube connected to the chemical liquid connecting apparatus; and
   a push-out liquid injection apparatus feeding the push-out liquid to the liquid supply tube.

### Effect of the Invention

According to the present invention, in the injection of the chemical liquid into a patient, the air can be removed efficiently from the liquid supply tube to the injection tube and the operator can be protected securely against injuries from sharp-pointed needles.

### Brief Description of the Drawings

Fig. 1 shows an example of a connection adapter.
Fig. 2 shows an example of the connection adapter.
Fig. 3 schematically shows the internal structure of the connection adapter.
Fig. 4 shows the connection adapter in use.
Fig. 5 schematically shows air removal.
Fig. 6 shows an example of a protection cap removing mechanism.
Fig. 7 shows the interior of the example of the protection cap removing mechanism.
Figs. 8 show an example of an engaging mechanism for use in the protection cap removing mechanism.
Fig. 9 is a diagram for explaining the use of the protecting cap removing mechanism.
Fig. 10 is a diagram for schematically explaining the engagement of the protection cap removing mechanism and a protecting cap.
Fig. 11 is a diagram for explaining the use of the protecting cap removing mechanism.
Fig. 12 shows an example of a chemical liquid connecting apparatus.
Fig. 13 schematically shows a medical imaging system as a whole.
Fig. 14 shows an example of a push-out liquid injection apparatus.
Fig. 15 is a perspective view showing the internal structure of the chemical liquid connecting apparatus.
Figs. 16 show the operation of the chemical liquid connecting apparatus.
Figs. 17 show the operation of the chemical liquid connecting apparatus.

### Description of Reference Numerals

- 10: ADAPTER
- 11: HOLDER PORTION
- 12: SUPPLY NEEDLE
- 13: DISCHARGE NEEDLE
- 14: LIQUID SUPPLY TUBE CONNECTION PORTION
- 15: CHEMICAL LIQUID INJECTION TUBE CONNECTION PORTION
- 20: PROTECTION CAP
- 22: ENGAGING CONVEX PORTION
- 30: REMOVING MECHANISM
- 31: SPACE PORTION
- 32: ENGAGING MECHANISM
- 100: PUSH-OUT LIQUID INJECTION APPARATUS
- 120: LIQUID SUPPLY TUBE
- 130: CHEMICAL LIQUID INJECTION TUBE
- 300: CONNECTING APPARATUS
- 301: CONNECTING APPARATUS BODY
- 310: CONTAINER MOVING MECHANISM
- 311: GUIDE RAILS
- 315: CONTAINER HOLDING MECHAINSM
- 320: HOLDER MOVING MECHANISM
- 329: THROUGH HOLE
- 330: CONTAINER LIFTING MECHANISM
- 331: TABLE
- 332: GUIDE SHAFT
- 400: SHIELD CONTAINER

### Best Mode for Carrying Out the Invention

### <Connection adapter including protection cap>

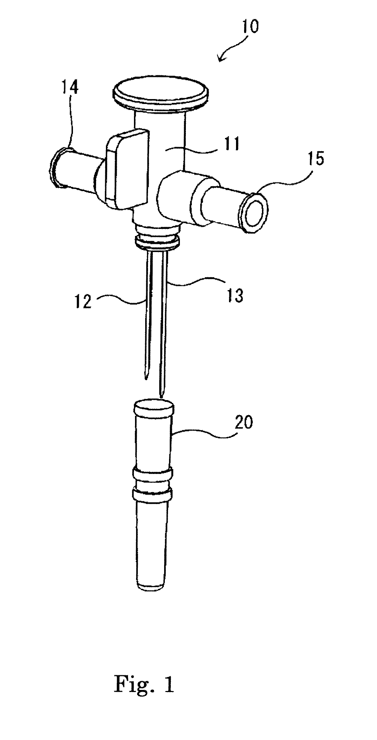

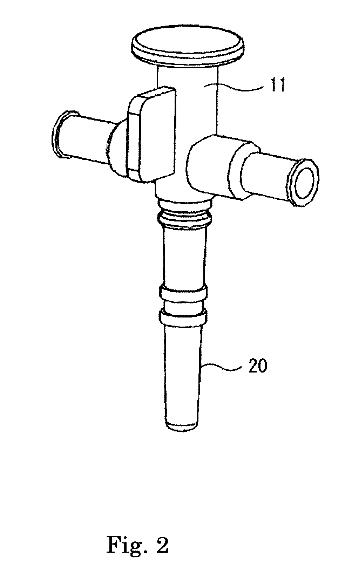

An example of a connection adapter according to the present invention will be described with reference to the accompanying drawings. As shown in Fig. 1, connection adapter 10 according to the present invention includes liquid supply tube connection portion 14, chemical liquid injection tube connection portion 15, supply needle 12, discharge needle 13, holder portion 11, and protection cap 20. Fig. 2 shows protection cap 20 fit on holder portion 11. Connection adapter 10 is commercially available with the protection against the needles provided in this manner.

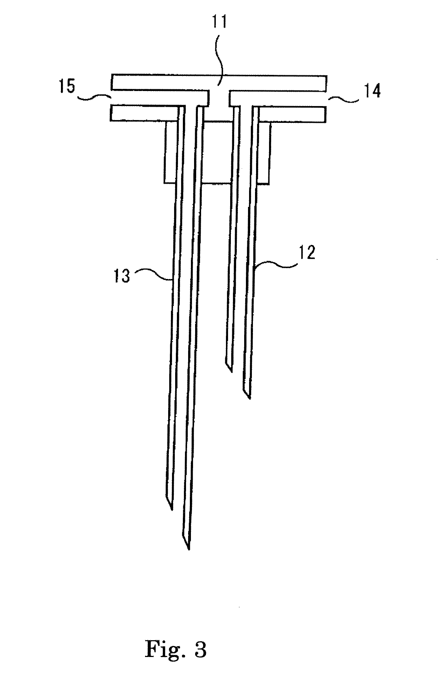

As shown in Fig. 3, in the connection adapter, supply needle 12 communicates with liquid supply tube connection portion 14, while discharge needle 13 communicates with chemical liquid injection connection portion 15. However, liquid supply tube connection portion 14 does not communicate with chemical liquid injection tube connection portion 15, and no liquid directly flows between them.

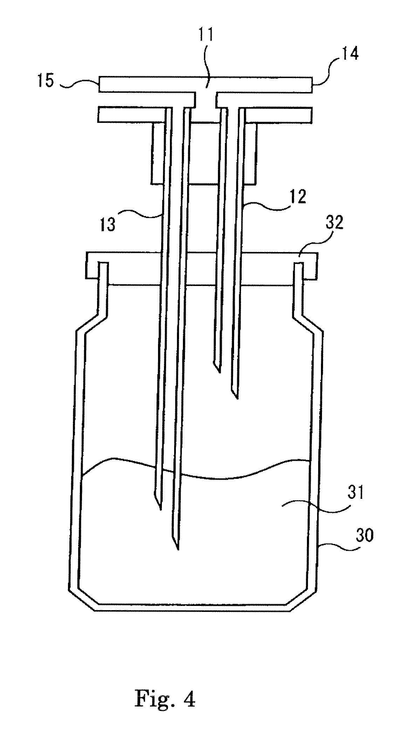

Fig. 4 shows the connection adapter in use. Chemical liquid container 30 contains chemical liquid 31 and has an opening at the top sealed with elastic member 32 made of silicone rubber or the like. When the connection adapter according to the present invention is used, needles 12 and 13 are inserted into chemical liquid container 30 through the elastic member. In this state, a push-out liquid is supplied into chemical liquid container 30 through a liquid supply tube (not shown) connected to connection portion 14. Then, chemical liquid 30 present in the chemical liquid container is pushed out and fed through discharge needle 13 into an injection tube (not shown) connected to connection portion 15 and thus the chemical liquid is injected into a patient. Chemical liquid 30 preferably contains a radioactive material for use in an RI test. Although described later in detail, exposure of an operator to radiation can be minimized by using a shield member and following an appropriate procedure.

For the injection of the chemical liquid into the patient, the liquid supply tube and the injection tube are connected to the connection adapter as described above. The liquid supply tube and the injection tube generally have a length equal to or more than one meter in total. Prior to the injection of the chemical liquid, it is necessary to remove air from the line including the tubes, a needle and a catheter and the like attached to the leading end of the injection tube. The connection adapter according to the present invention is used significantly effectively for removing air from the line.

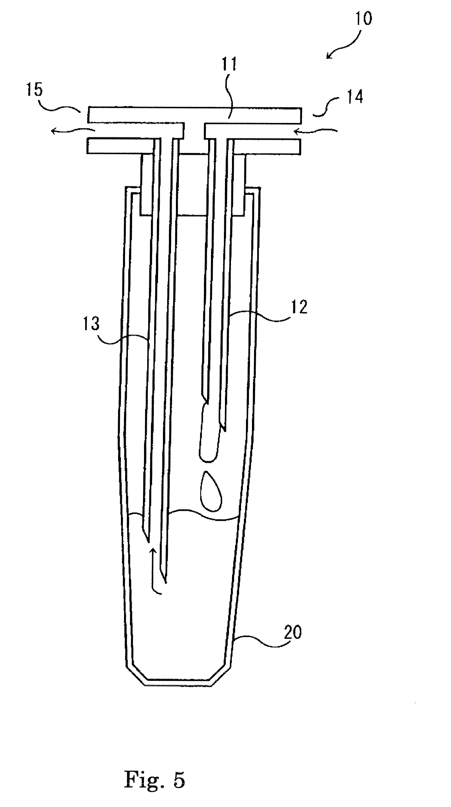

Fig. 5 schematically shows the air removal in connection adapter 10. Protection cap 20 is fit on holder portion 11 such that needles 12 and 13 are placed in the seal space within protection cap 20. When the push-out liquid is supplied in this state, the push-out liquid (physiological saline) is filled into protection cap 20 through liquid supply tube connection portion 14 and supply needle 12. Then, the push-out liquid is discharged through discharge needle 13 while removing the air within the protection cap. The push-out liquid can pass through injection tube connection portion 15 and fill the line from the injection tube onward.

After the push-out liquid (physiological saline) fills the line in this manner, the protection cap is removed and the connection adapter is mounted on the chemical liquid container as shown in Fig. 4. As a result, subsequent air removal is hardly required or is necessary only as a simple check, so that chemical liquid 31 can be injected into a patient easily and conveniently.

### <Protection cap removing mechanism>

In another aspect of the present invention, a protection cap removing mechanism is provided for easily removing the protection cap. The protection cap may be manually removed as described above. However, the protection cap is preferably removed with the protection cap removing mechanism in order to prevent the operator from being injured by the needle and to adapt to an apparatus later described.

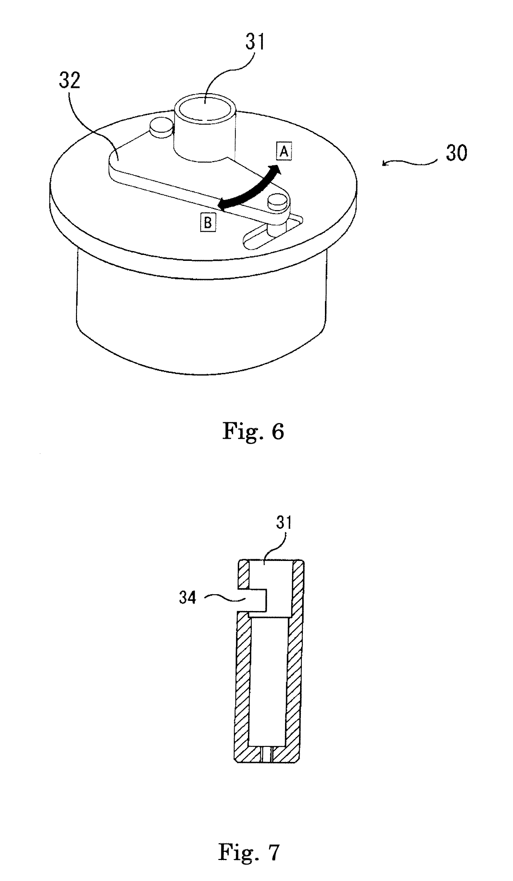

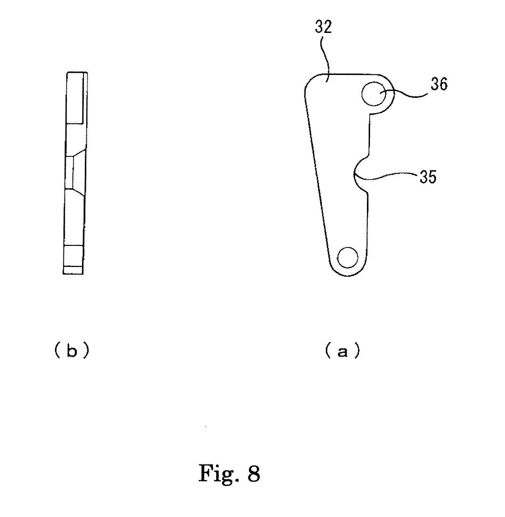

Fig. 6 shows an example of the protection cap removing mechanism (hereinafter referred to simply as a removing mechanism). Removing mechanism 30 of the example includes space portion 31 which can accommodate at least a lower portion of the protection cap and engaging mechanism 32. Except its upper portion, removing mechanism 30 has a cylindrical outer shape similar to the shape of a shield container to adapt to the apparatus later described. Fig. 7 shows space portion 31 in detail. Space portion 31 has a cylindrical shape for accommodating the lower portion of the protection cap and has notch 34 formed therein to allow an engaging piece of the engaging mechanism 32 to extend into space portion 31. Figs. 8 show engaging mechanism 32 in detail. As shown in Fig. 8(b), the engaging mechanism has a flat shape as a whole and can pivot about axis 36. Portion 35 for engagement with the protection cap is preferably formed in an arc shape as shown.

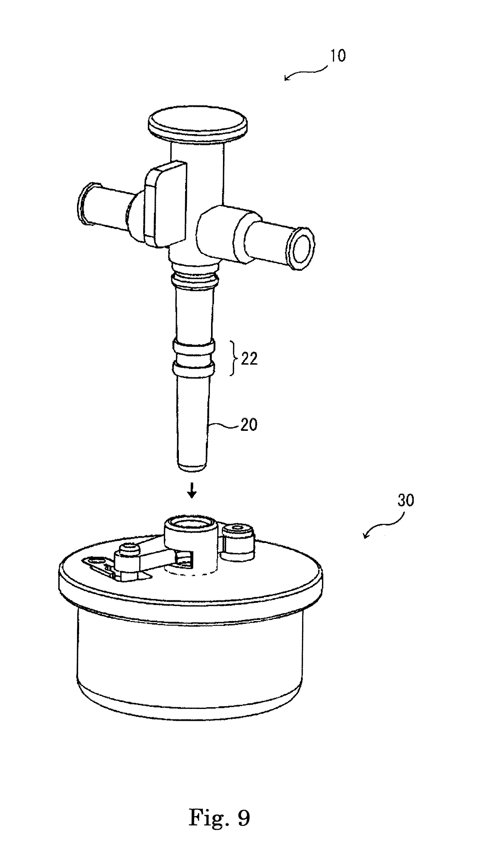

For use of removing mechanism 30, the connection adapter with the protection cap fit thereon is inserted into space portion 31 from above as shown in Fig. 9. The protection cap preferably has strip-shaped engaging convex portion 22. In the insertion, engaging mechanism 32 is pivoted in direction B shown in Fig. 6 to allow the insertion of the protection cap. After the insertion, engaging mechanism 32 is switched to direction A. Alternatively, the engaging mechanism may be urged to direction A such that it may be opened in direction B for insertion and returned to direction A after the insertion.

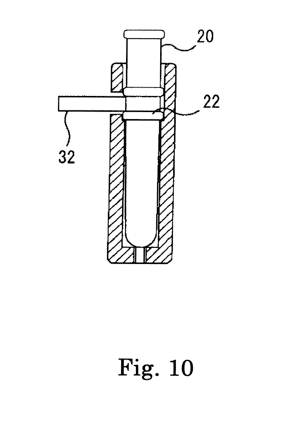

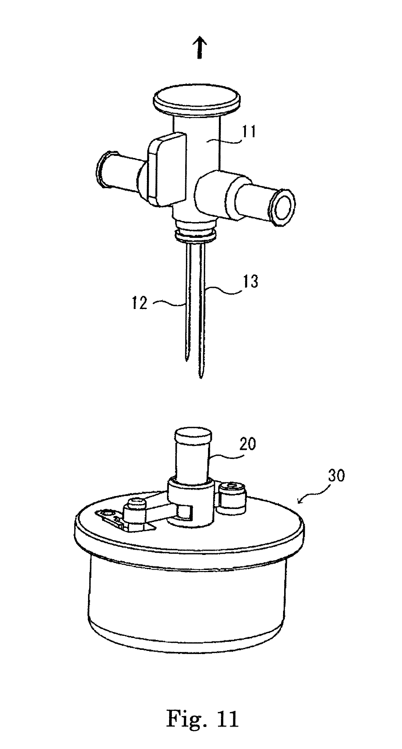

Fig. 10 shows the protection cap inserted into space portion 31 such that engaging mechanism 32 engages with engaging portion 22 (the convex portion in Fig. 10) of protection cap 20. In this state, holder portion 11 of the connection adapter is raised. Then, as shown in Fig. 11, protection cap 20 is removed and left in removing mechanism 30 to expose supply needle 12 and discharge needle 13. After the protection cap is removed safely in this manner, the two needles are inserted into elastic member 32 of chemical liquid container 30 as described above and the injection can be started. The air removal can be performed at any time without particular limitations while the protection cap is fit on holder portion 11. However, the air removal is preferably performed typically at the time when the connection adapter with the protection cap fit thereon is mounted on space portion 31 of removing mechanism 30, as later described.

### <Chemical liquid connecting apparatus, Chemical liquid injector>

Next, description will be made for a chemical liquid connecting apparatus including the connection adapter having the protection cap and the protection cap removing mechanism, and a medical imaging apparatus according to the present invention.

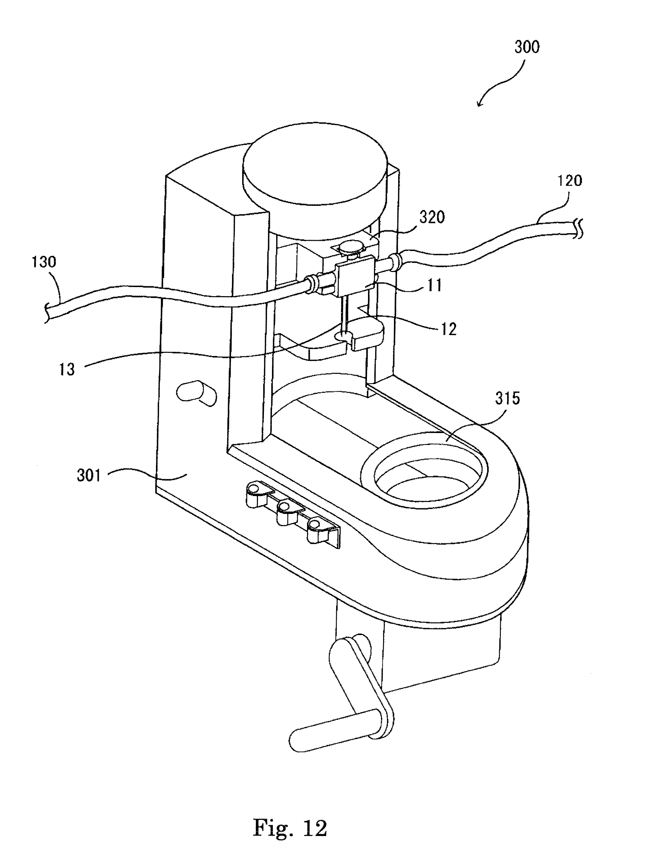

As shown in Figs. 12 to 14, medical imaging system 1000 to which the present invention is applied includes connecting apparatus 300, the chemical liquid container (not shown), shield container 400, push-out liquid injection apparatus 100, a syringe (not shown) mounted on injector 100, and PET apparatus 500 serving as a diagnostic imaging apparatus. The push-out liquid (for example, physiological saline) is supplied to the chemical liquid container from push-out liquid injection apparatus 100, a radioactive contrast medium containing a radioisotope is injected into a patient (not shown), and diagnostic images of the patient are shot by PET apparatus 500.

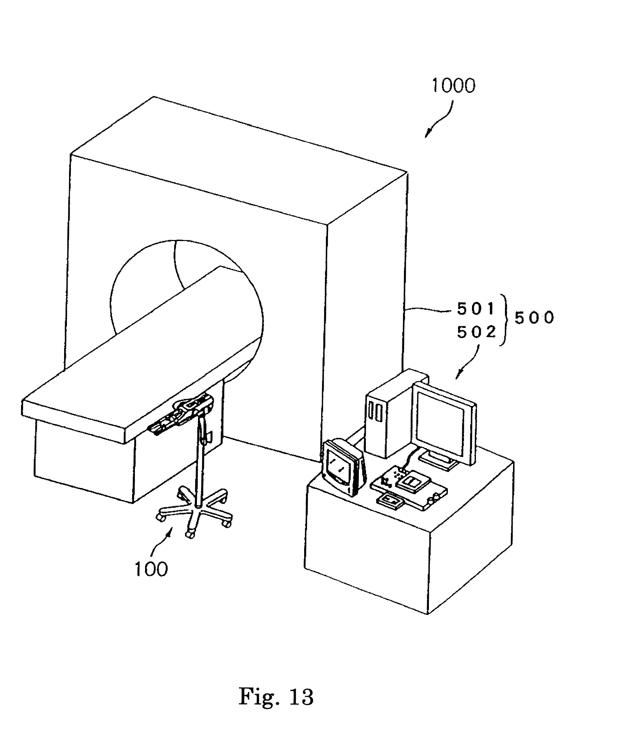

As shown in Fig. 13, PET apparatus 500 includes imaging diagnostic unit 501 serving as an imaging means and imaging control unit 502 such that imaging diagnostic unit 501 and imaging control unit 502 are wire-connected. Imaging diagnostic unit 501 shoots diagnostic images of the patient. Imaging control unit 502 controls the operation of imaging diagnostic unit 501.

The chemical liquid injector according to the present invention is preferably used in the abovementioned medical imaging system and the like, and includes not only push-out liquid injection apparatus 100 and chemical liquid connecting apparatus 300 but also tubes for connecting push-out liquid injection apparatus 100 to chemical liquid connecting apparatus 300 and tubes for feeding the chemical liquid into the patient from chemical liquid connecting apparatus 300.

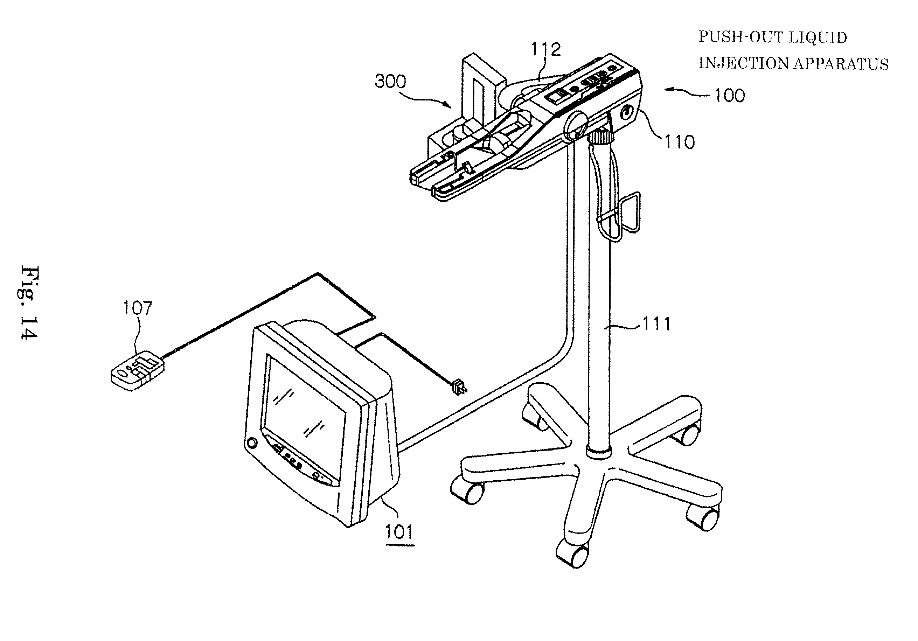

As shown in Fig. 14, push-out liquid injection apparatus 100 includes injection head 110 supported on the top end of caster stand 111 by movable arm 112, and injection control unit 101 and control unit 107 and the like as separate components from injection head 110. A syringe filled with a liquid for supply such as physiological saline is mounted on injection head 110. The push-out liquid is fed into liquid supply tube 120 by moving a syringe piston relative to a cylinder. In general, the liquid for supply is preferably a liquid having a specific gravity lower than that of a contrast medium and should be harmless to human bodies. Typically, physiological saline is preferably used.

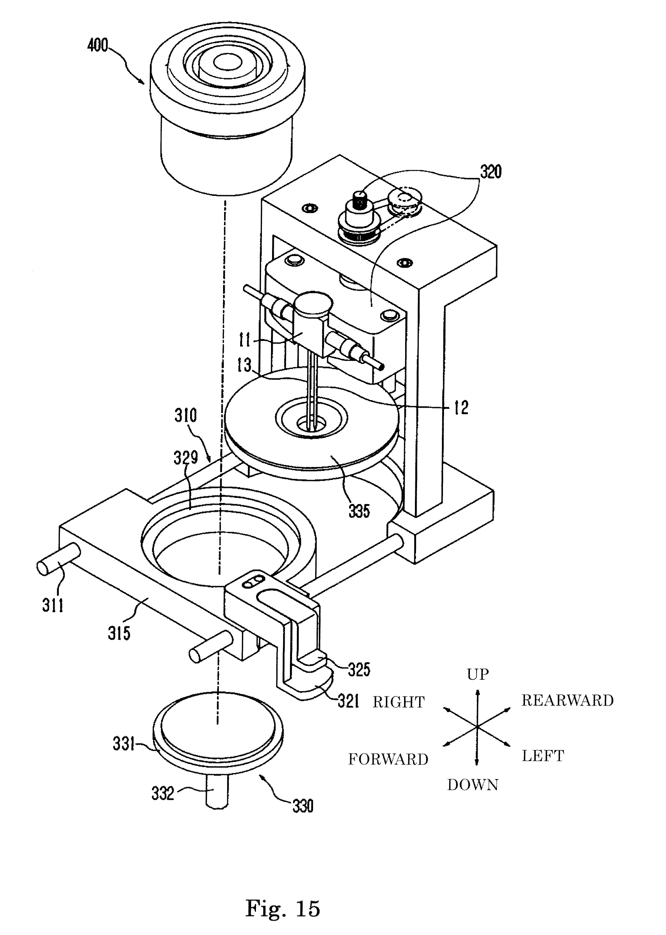

As shown in Figs. 12 and 15, chemical liquid connecting apparatus 300 of the embodiment has L-shaped connecting apparatus body 301 which includes container moving mechanism 310 in its lower portion elongated in a front-back direction. As shown in Fig. 15, container moving mechanism 310 is embodied by a slider structure including guide rails 311 and a screw mechanism and supports container holding mechanism 315 to be movable in the front-back direction. In the structure in which container holding mechanism 315 is manually moved, it has move guide knob 321, lock release mechanism 325 and the like as shown in Fig. 15. When container holding mechanism 315 is moved through mechanical driving, it has a DC motor (not shown), and a position detecting means and the like as required.

Container holding mechanism 315 may have a shape capable of holding shield container 400 which accommodates the chemical liquid container, and for example, it preferably includes through hole 329 and container lifting mechanism 330. The container lifting mechanism includes table 331 for holding shield container 400 from below, guide shaft 332 and the like.

Chemical liquid connecting apparatus 300 also has holder moving mechanism 320 for lifting holder 11 of connection adapter 10 up and down. The function of the holder moving mechanism allows the removal of the protection cap and the insertion of the two needles into the elastic member of the chemical liquid container.

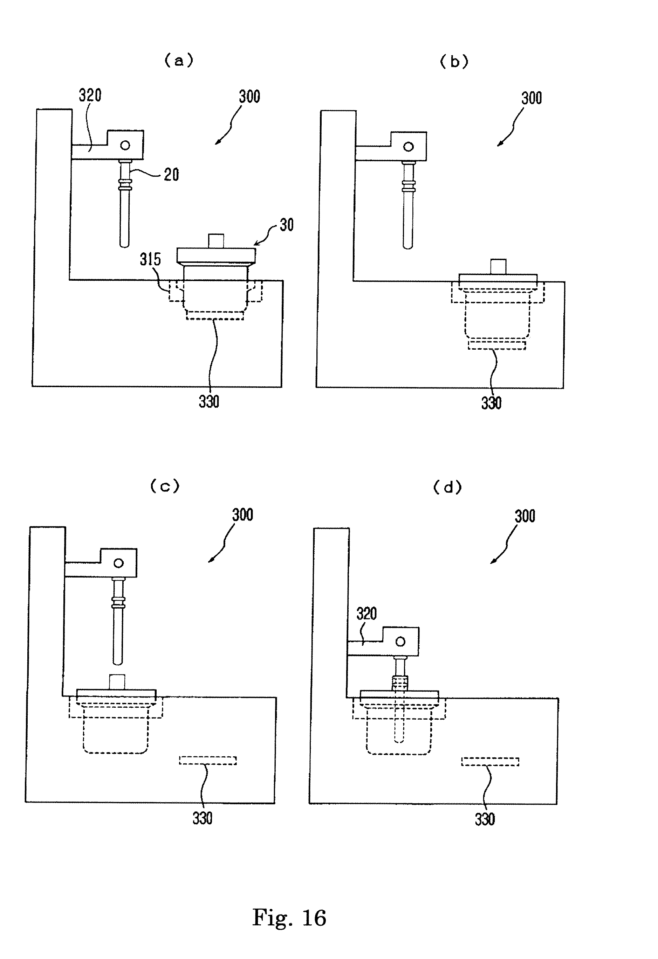

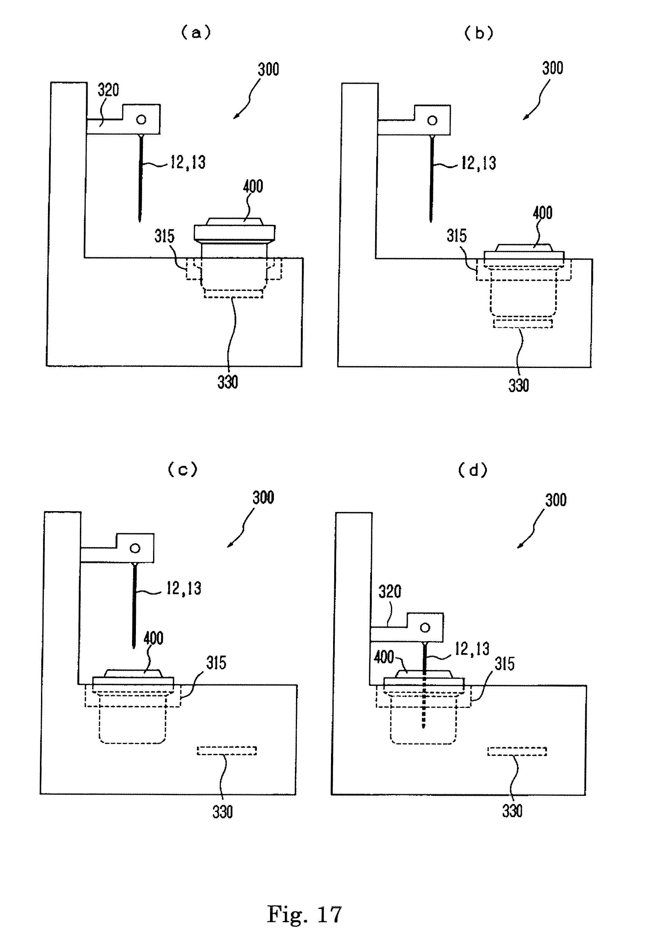

Next, the functions of the respective members will be described in detail with reference to the operation procedures of the chemical liquid connecting apparatus and the chemical liquid injector.
1) Connection adapter 10 with protection cap 20 fit thereon is prepared. Liquid supply tube 120 and chemical liquid injection tube 130 are connected to connection portions 14 and 15, respectively. Connection adapter 10 is attached to holder moving mechanism 320 of connecting apparatus 300.
2) A syringe filled with physiological saline is attached to the trailing end of liquid supply tube 120 and is mounted on push-out liquid injection apparatus 100. A needle such as a butterfly needle or a catheter is connected to the leading end of the chemical liquid injection tube for injecting the chemical liquid into the patient. The orders of the attachment and the connection in 1) and 2) are not essential.
3) Removing mechanism 30 is put on container holding mechanism 315 as shown in Fig. 16(a). Next, as shown in Fig. 16(b), container lifting mechanism 330 is manually or automatically lowered. When container lifting mechanism 330 is completely lowered, container holding mechanism 315 can be moved in the front-back direction. As shown in Fig. 16(c), container holding mechanism 315 is moved manually or automatically directly below the connection adapter with protection cap 20 fit thereon, that is, to an insertion position. Next, as shown in Fig. 16(d), holder moving mechanism 320 is lowered to cause protection cap 20 to be placed in space portion 31 of removing mechanism 30. Then, engaging mechanism 32 engages with protection cap 20.
4) With protection cap 20 fit on holder portion 11, push-out liquid injection apparatus 100 is operated to fill the physiological saline from liquid supply tube 120 to the leading end of chemical liquid injection tube 130 to remove the air as described in the section of <Connection adapter including protection cap>. The air removal may be performed at any time while the protection cap is fit on holder portion 11.
5) After the air removal is completed, holder moving mechanism 320 is raised. As shown in Fig. 11, holder portion 11 is raised with protection cap 20 left in removing mechanism 30 to expose supply needle 12 and discharge needle 13. Then, removing mechanism 30 is manually or automatically returned to the initial state in the order of the steps of Fig. 16(c), 16(b), and 16(a), and is removed from container holding mechanism 315 with protection cap 20 placed in space portion 31. In this manner, the air removal in the line and the removal of the protection cap are finished.
6) Next, the chemical liquid container housed in shield container 400 made of a radiation shield material such as tungsten is prepared and put on container holding mechanism 315 of connecting apparatus 300. When the top lid of shield container 400 is opened, the elastic member of silicone rubber or the like for sealing the top opening of the chemical liquid container is exposed. Fig. 17(a) shows this state. Next, as shown in Fig. 17(b), container lifting mechanism 330 is manually or automatically lowered. After container lifting mechanism 330 is completely lowered, container holding mechanism 315 can be moved in the front-back direction. As shown in Fig. 17(c), container holding mechanism 315 is moved manually or automatically directly below the connection adapter with supply needle 12 and discharge needle 13 exposed. Next, as shown in Fig. 17(d), holder moving mechanism 320 is lowered to insert supply needle 12 and discharge needle 13 into the elastic member.
7) In this state, push-out liquid injection apparatus 100 is operated to inject the physiological saline into the chemical liquid container as described with reference to Fig. 4. In general, the supply needle is shorter than the discharge needle and the physiological saline is lighter than the chemical liquid containing a radioactive source, so that the supplied physiological saline pushes and injects the chemical liquid into the patient through the discharge needle. A sufficient amount of the push-out liquid such as physiological saline is used for discharging the chemical liquid. Even when the chemical liquid diluted with the push-out liquid is injected, no problem occurs in RI diagnoses such as PET diagnoses.
8) Then, shield container 400 is returned through the operations in the reverse order of Fig. 17(d), 17(c), 17(b), and 17(a). In this manner, the injection of the chemical liquid preferably containing the radioactive source is finished.
9) In a preferable embodiment, removed protection cap 20 is again fit on the used connection adapter. Removing mechanism 30 taken away in the abovementioned step 5) is placed instead of shield container 400 in Fig. 17(a). At this point, removing mechanism 30 holds protection cap 20 in space portion 31. Supply needle 12 and discharge needle 13 are housed in protection cap 20 through the same procedure as that of Figs. 17(b) to 17(d). Then, engaging mechanism 32 is pivoted in direction B in Fig. 6 to release the engagement, and in this state, holder moving mechanism 320 is raised to return to the upper retraction position with protection cap 20 fit again on holder portion 11. Then, the protection cap is again mounted on connection adapter 10 through the procedure in the order of Fig. 17(d), 17(c), 17(b), and 17(a) (or Fig. 16(d), 16(C), 16(b), and 16(a)). Since the cap is not manually put on the needle in this manner, the used connection adapter is returned extremely safely. The operations involve a very limited possibility of contamination, so that the connection adapter according to the present invention can be reused.

Subsequent to the injection of the radioactive chemical liquid into the patient in this manner, the PET test is performed after the lapse of a predetermined time period.

The abovementioned operation procedure can be performed manually or automatically by using a control apparatus, a detection apparatus, and a driving apparatus as required.

In the above description, after the protection cap is removed, the removing mechanism with the protection cap placed therein is returned to the initial position of the container moving mechanism and is replaced with the shield container. However, the removing mechanism may be retracted to a temporary retraction position in a direction different from the initial position (for example, a perpendicular direction) by means of a similar slide mechanism or the like. In this case, it is unnecessary to replace the shield container with the removing mechanism when the protection cap is again put.

## Claims

1. A connection adapter comprising:
a liquid supply tube connection portion;
a chemical liquid injection tube connection portion;
a hollow supply needle communicating with the liquid supply tube connection portion;
a hollow discharge needle communicating with the chemical liquid injection tube connection portion;
a holder portion supporting the supply needle and the discharge needle; and
a protection cap removably fit on the holder portion, the supply needle and the discharge needle being accommodated by and hermetically sealed in the protection cap and the holder portion.

2. The connection adapter according to claim 1, wherein the connection adapter is used in a chemical liquid injection system in which the connection adapter is connected to a liquid supply tube and a chemical liquid injection tube with the protection cap being fit, and the connection adapter is supplied with a liquid from the liquid supply tube to remove air in the liquid supply tube and the chemical liquid injection tube, and then the protection cap is removed, and then the supply needle and the discharge needle are inserted into an elastic member of a chemical liquid container having an opening sealed with the elastic member and containing a chemical liquid, and a push-out liquid is supplied from the liquid supply tube into the chemical liquid container through the supply needle, whereby the chemical liquid in the chemical liquid container is discharged through the discharge needle, passes through the chemical liquid injection tube, and is injected into a patient.

3. The connection adapter according to claim 2, wherein the protection cap has a sheath shape elongated along the direction of the two needles and has an engaging portion formed in a part of the sheath shape.

4. A protection cap removing mechanism for the connection adapter according to any one of claims 1 to 3, comprising:
a space portion opened upward and capable of accommodating a part of the protection cap from above; and
an engaging mechanism engaging with the protection cap placed in the space portion and preventing an upward movement of the protection cap when the holder portion of the connection adapter is moved upward.

5. The protection cap removing mechanism according to claim 4, wherein the space portion has a cylindrical shape and has a notch formed in a wall thereof, the engaging mechanism which has an engaging piece is moved in a direction perpendicular to a direction in which the protection cap is inserted and capable of extending into the space portion.

6. A chemical liquid connecting apparatus comprising:
the connection adapter according to any one of claims 1 to 3;
a container holding mechanism holding the chemical liquid container such that the opening of the container is placed at the top;
a holder moving mechanism vertically moving the connection adapter to an upper retraction position and a lower insertion position; and
a container moving mechanism horizontally moving the container holding mechanism to an insertion position opposed to the retraction position of the connection adapter in the under side and an initial position not opposed to the retraction position.

7. The chemical liquid connecting apparatus according to claim 6, further comprising the protection cap removing mechanism according to claim 4 or 5 and capable of being placed on the container moving mechanism.

8. The chemical liquid connecting apparatus according to claim 6 or 7, further comprising a container lifting mechanism vertically moving the chemical liquid container at the initial position.

9. The chemical liquid connecting apparatus according to claim 8, wherein the horizontal movement of the container moving mechanism is allowed at the position where the container lifting mechanism is lowered at the initial position.

10. The chemical liquid connecting apparatus according to any one of claims 6 to 9, wherein the chemical liquid is a contrast medium containing a radioactive source, and the chemical liquid container is housed in a shield container and is placed in the container holding mechanism with the elastic member of the chemical liquid container exposed at the top.

11. A chemical liquid injector comprising:
the chemical liquid connecting apparatus according to any one of claims 6 to 10;
a liquid supply tube connected to the chemical liquid connecting apparatus;
a chemical liquid injection tube connected to the chemical liquid connecting apparatus; and
a push-out liquid injection apparatus feeding the push-out liquid to the liquid supply tube.
